# EUROPÄISCHE PATENTSCHRIFT

(11) **EP 1 240 107 B1**
(45) Veröffentlichungstag und Bekanntmachung des Hinweises auf die Patenterteilung: **09.07.2003**
(21) Anmeldenummer: 00969564.4
(22) Anmeldetag: 28.10.2000
(51) Int. Cl.: C02F 1/42, C07C 51/47

(54) **VERFAHREN ZUR RÜCKGEWINNUNG FLUORIERTER EMULGATOREN**
METHOD FOR RECOVERING FLUORINATED EMULSIFIERS
PROCEDE DE RECUPERATION D'EMULSIFIANTS FLUORES

(30) Priorität: 05.11.1999 DE 19953285
(43) Veröffentlichungstag der Anmeldung: 18.09.2002
(73) Patentinhaber: Dyneon GmbH & Co. KG, 84504 Burgkirchen (DE)
(72) Erfinder: FÜHRER, Stephan, 84556 Kastl (DE); HINTZER, Klaus, 84556 Kastl (DE); LÖHR, Gernot, 84508 Burgkirchen (DE); SCHWERTFEGER, Werner, 84503 Altötting (DE)
(74) Vertreter: Voortmans, Gilbert J.L.
(86) Internationale Anmeldenummer: EP0010638
(87) Internationale Veröffentlichungsnummer: WO01032563

(56) Entgegenhaltungen:
- EP-A- 0 014 431
- DE-A- 4 318 258
- US-A- 3 882 153

## Beschreibung

Die Erfindung betrifft ein Verfahren zum Eluieren fluorierter Emulgatoren aus Anionenaustauscherharzen im basischen Milieu, das besonders nützlich zur Reinigung von Abwasser ist, das aus der Herstellung von Fluorpolymeren stammt.

Fluorpolymere, wie Fluorelastomere, Fluorthermoplaste und Polytetrafluorethylen (PTFE) werden durch wäßrige radikalische Emulsionspolymerisation hergestellt. Die Emulsionspolymerisation erfordert hochfluorierte Emulgatoren, um eine zufriedenstellende Kolloidstabilität zu gewährleisten. Die verwendeten Emulgatoren sollen nicht mit den Fluorpolymerradikalen reagieren und deshalb nicht telogen sein. Üblicherweise verwendet man APFOS, das Ammoniumsalz der Perfluoroctansäure (PFOS).

Auf die Polymerisation folgt das Isolieren des Polymers durch Koagulieren der Polymerdispersion entweder mechanisch bei hoher Schergeschwindigkeit oder chemisch durch Zugabe starker Mineralsäuren wie HCl oder HNO₃. Die koagulierten Fluorthermoplaste werden gewöhnlich agglomeriert, indem organische, meist mit Wasser nicht mischbare Lösungsmittel, zum Beispiel Benzin oder fluorierte Kohlenwasserstoffe, zugesetzt werden. Die isolierten Polymerharze werden mit Wasser gewaschen. Mit 5 bis 10 t Wasserverbrauch pro Tonne Harz fallen relativ große Mengen Prozeßwasser an, das hier als Abwasser bezeichnet wird. Das Abwasser enthält im Mittel 30 bis zu 1000 ppm PFOS. Charakteristisch für die Koagulation von in Gegenwart fluorierter Emulgatoren hergestellten Fluorpolymeren ist, daß der Emulgator bei der Koagulation in hohem Maße desorbiert und zu einem sehr großen Anteil ausgewaschen wird. So gelangen mehr als 80 % des benutzten fluorierten Emulgators ins Abwasser. Dieses Abwasser enthält neben Latexteilchen die Hilfsmittel der verschiedenen Polymerisationsrezepte wie Puffer, Initiatoren und verschiedene Kettenüberträger, zum Beispiel Diethylmalonester und seine Zersetzungsprodukte, Alkylchloride oder Alkane, Benzin und andere organische Verbindungen aus der Aufarbeitung.

Der fluorierte Emulgator, insbesondere APFOS, ist eine sehr teure Verbindung und trägt sehr beträchtlich zu den Gesamtkosten der Fluorpolymere aus der Emulsionspolymerisation bei. Folglich ist aus ökonomischen Gründen seine Rückgewinnung sehr bedeutsam. Darüber hinaus sind fluorierte Emulgatoren biologisch praktisch nicht abbaubar und nach jüngsten Untersuchungen besteht der Verdacht einer Gesundheitsgefährdung.

Deshalb ist es wünschenswert, den Emulgator aus dem Abwasser zu entfernen, wieder zurückzugewinnen und in die Emulsionspolymerisation zurückzuführen. Die Entfernung aus dem Abwasser erfolgt vorzugsweise mit Anionenaustauscherharzen gemäß US-A-3 882 153 und EP-B-14 431. Bezüglich dieser Angelegenheit wurden weitere Abwasserbehandlungen in WO-A-99/62858 und WO-A-99/62830 vorgeschlagen. Beide Abwasserbehandlungen betreffen die Entfernung von PFOS aus industriellen Abwässern über Anionenaustauscher, wobei stark basische Anionenaustauscher empfohlen werden. Der Anionenaustausch ist behindert durch die Anwesenheit von Fluorpolymer-Latexpartikeln, die die Anionenaustauscherkolonnen verstopfen. Gemäß WO-A-99/62858 werden die Latexpartikel durch Zugabe von relativ hohen Salzmengen vor dem Ionenaustausch ausgefällt. Nach WO-A-99/62830 werden die feinen Latexpartikel durch den Zusatz von nichtionischen Tensiden stabilisiert, und man verhindert dadurch, selbst bei Dauerbetrieb, das Verstopfen der Anionenaustauscherkolonne.

Die Entfernung des Emulgators aus dem Abwasser mit einem Anionenaustauscherharz ist sehr effizient. Insbesondere mit stark basischen Anionenaustauscherharzen erfolgt eine praktisch quantitative Entfernung. Der Emulgator wird selektiv adsorbiert und somit kann die gesamte Kapazität des Ionenaustauschers ausgenutzt werden.

Aber entsprechend der starken Adsorption ist das Eluieren, im folgenden als Zurückgewinnen bezeichnet, erheblich erschwert. Beispielsweise ergibt das Eluieren eines mit bis zum Durchbruch mit APFOS beladenen stark basischen Anionenaustauscherharzes mit jeweils Lösungen von 1 Mol/l NH₃, NaOH und KF APFOS-Konzentrationen im Eluat im Bereich von lediglich 0,1 mMol/l (40 ppm). Folglich ist dieses Verfahren zum Zurückgewinnen von APFOS aus größeren Abwasservolumina ökonomisch nicht vertretbar.

Zum Eluieren für eine wirtschaftliche Rückführung ist eine möglichst hohe Eluat-Peak-Konzentration erforderlich. Nach EP-B-14 431 ist eine Spitzenkonzentration von 180 000 ppm mit einem Eluens aus 89 Gew.-% Methanol, 4 Gew.-% Schwefelsäure und 7 Gew.-% Wasser erzielbar. Die Elution erfolgt im sauren Milieu.

Im basischen Milieu wird nach US-A-3 882 153 mit einem APFOS beladenen, schwachbasischen Anionenaustauscher beim Eluieren mit 0,5 bis 2 molarer wäßriger Ammoniaklösung eine relativ hohe Peak-Konzentration erzielt. Jedoch ist die erzielbare Peak-Konzentration von APFOS um den Faktor 2 bis 3 niedriger als in EP-B-14 431 und für einen technisch praktikablen Prozeß noch nicht zufriedenstellend.

Nach der nicht vorveröffentlichten deutschen Patentanmeldung 199 32 771.8 vom 14.07.1999 verläuft die Elution stark basischer Anionenaustauscherharze im basischen Milieu in Anwesenheit von mit Wasser mischbaren organischen Lösungsmittel wesentlich effizienter. Jedoch wird die Peak-Konzentration von 180 000 der EP-B-14 431 nur mit relativ technisch umständlichen Mischungen erreicht.

Es ist die Aufgabe der vorliegenden Erfindung, ein Verfahren bereitzustellen, bei dem fluorierte Emulgatoren, insbesondere PFOS, aus Anionenaustauschern mit technisch einfachen Eluensmischungen bei möglichst hohen APFOS-Konzentrationen zurückgewonnen werden können.

Es wurde nun gefunden, daß bei mittel- und schwachbasischen Anionenaustauscherharzen die PFOS-Konzentrationen im Eluat mit ammoniakalischem organischem Lösemittel erheblich gesteigert werden kann. In Gegenwart von Alkalihydroxiden wird diese Eluatkonzentration noch weiter gesteigert.

Als Lösung dieser Aufgabe wird ein Verfahren zum Eluieren fluorierter Emulgatoren bereitgestellt, bei dem mit fluorierten Emulgatoren, insbesondere PFOS beladene, schwach bis mittelstark basische Anionenaustauscherharze mit Ammoniak enthaltenden, mit Wasser mischbaren organischen Lösemitteln eluiert werden.

Erfindungsgemäß können die im Stand der Technik erreichbaren 180 000 ppm deutlich übertroffen werden, zum Beispiel über 300 000 ppm, und zwar mit technisch einfachen Eluensmischungen.

Die vorliegende Erfindung ermöglicht bedeutend höhere Spitzenkonzentrationen und verringert damit beträchtlich die Menge des für die Regenerierung eines Anionenaustauscherharzes benötigten, leicht entflammbaren organischen Lösungsmittels.

Für die technische Handhabung der Rückgewinnung bringen solch konzentrierte Eluate erhebliche Vorteile. Desweiteren wird die Rückführung der PFOS erheblich erleichtert, wie im folgenden ausgeführt wird: Das organische Lösungsmittel, einschließlich dem Ammoniak, wird destillativ abgetrennt und gegebenenfalls durch Wasser ersetzt. Die Destillatmischung kann unmittelbar zur Regenerierung des Ionenaustauschers benutzt werden. Bei der Destillation werden aus dem Eluat die organischen Verunreinigungen aus dem Abwasser, wie Kettenüberträger oder Benzin, aus der Aufarbeitung beim Entfernen des organischen Lösungsmittels problemlos entfernt, da die Salze der PFOS nicht wasserdampfflüchtig sind.

Die vorliegende Erfindung stellt ein technisches Verfahren zur Rückgewinnung der PFOS aus industriellen Abwässern dar. Recycling im Sinne der vorliegenden Erfindung besteht in der Bereitstellung des Emulgators, der frei von in die Polymerisation eingreifenden Verunreinigungen ist.

Die Ammoniakkonzentration in der Mischung beträgt vorteilhaft mindestens 0,1 mol/l und höchstens 4 mol/l, vorzugsweise 1 bis 2,5 mol/l. Die Ammoniakkonzentration wird zweckmäßigerweise durch Zugabe von entsprechenden Mengen wäßriger konzentrierter Ammoniaklösungen, wie im Handel erhältlich, zu dem organischen Lösemittel hergestellt. Das System muß eine hinreichende Menge Wasser enthalten, damit die erforderlichen Anionen freigesetzt werden können.

Geeignete organische Lösungsmittel sind mit Wasser mischbar und sollten einen Siedepunkt unter 150 °C aufweisen, vorzugsweise unter 110 °C. Unter "mischbar" wird hierbei vorzugsweise "unbegrenzt mischbar" verstanden. Bevorzugte Lösemittel, die einzeln oder im Gemisch eingesetzt werden können, sind Alkanole mit 1 bis 4 Kohlenstoffatomen, Aceton, Dialkylether des Monoglykols und Diglykols, wobei hier unter Alkylgruppen Methyl oder Ethyl verstanden wird. Besonders bevorzugte Lösemittel sind Methanol, Ethanol, n- und iso-Propanol und Dimethylmonoglykolether. Das unerläßliche Kation ist das Ammoniumion. Eine Zugabe von Alkalihydroxiden führt zu höheren Eluatkonzentrationen.

Die Definition für starke, mittlere und schwachbasische Anionenaustauscher findet sich in "Encyclopedia of Polymer Science and Engineering", John Wiley & Sons, 1985, Band 8, Seite 347 und "Kirk-Othmer", John Wiley & Sons, 3. Ausgabe, Band 13, Seite 687. Stark basische Anionenaustauscher enthalten typischerweise quartäre Amine, mittelstarke tertiäre Amine und die schwachen sekundären Amine als funktionelle Austauschstellen. Die Harzlieferanten klassifizieren die Stärke der Harze in den Produktbeschreibungsbeilagen.

Bevorzugt sind mittelstarke Anionenaustauscher aufgrund ihrer guten Wirksamkeit beim Entfernen von APFOS aus industriellen Abwässern, insbesondere in Gegenwart von in der deutschen Patentanmeldung 199 33 696.2 vom 17.07.1999 beschriebenen nichtionischen Emulgatoren.

Dieses erfinderische Verfahren ist grundsätzlich mit allen fluorierten anionischen Emulgatoren durchführbar. Es betrifft im wesentlichen fluorierte Alkancarbon- und Alkansulfonsäuren, wobei der Alkylrest teilweise oder vorzugsweise vollständig fluoriert ist und im allgemeinen linear oder auch verzweigt ist.

Unter dem Begriff fluorierte Emulgatoren sollen hier vor allem perfluorierte Alkansäuren der Formel CF₃(CF₂)ₙCOOH (n = 3 bis 10), insbesondere PFOS, verstanden werden, auf die das erfindungsgemäße Verfahren bevorzugt anwendbar ist. Unter diesen Begriff fallen aber auch teilfluorierte Alkansäuren der Formel XCF₂(CF₂)ₙCOOH (X = H oder Cl, n = 3 bis 10), perfluorierte oder teilfluorierte Alkansulfonsäuren der Formel XCF₂(CF₂)ₙSO₃H (X = H oder vorzugsweise F, n = 3 bis 10) sowie Perfluor-[(β-propoxy)propionsäure]. Der fluorierte Emulgator kann auch [(CF₂)ₙ-O]-Gruppen enthalten mit n ≥ 2. Auch diese fluorierten Emulgatorsäuren können nach dem erfindungsgemäßen Verfahren eluiert werden. Es können auch Gemische der genannten fluorierten Emulgatorsäuren adsorbiert und eluiert werden, insbesondere solche, die PFOS als Hauptbestandteil enthalten.

### Beispiele

Säulen einer Länge von 30 cm und einem Durchmesser von 6,5 cm werden mit 200 bis 240 ml des mittelstarken Ionenaustauschers ®Amberlite IRA 67 oder des schwachen Anionenaustauscher ®Amberlite IRA 92 befüllt, beide vertrieben von Rohm und Haas GmbH, Deutschland. Diese Austauscherharze werden mit APFOS, erhältlich von 3M unter dem Markennamen ®FC 143, bis zum Durchbruch beladen. Die beladenen Austauscherharze werden mindestens mit dem 5-fachen Bettvolumen an deionisiertem Wasser gewaschen. Die PFOS-Konzentration liegt im letzten Waschwasser unter 30 ppm. Darauf erfolgt die Eluierung mit Mischungen aus Methanol und einer entsprechenden Menge 28 Gew.-% wäßriger Ammoniaklösung bei einer Eluiergeschwindigkeit von 100 ml/h, in den Vergleichsbeispielen mit 1 Mol/l wäßriger Ammoniaklösung ohne Methanol. Die APFOS-Konzentrationen werden von jedem durchgelaufenen Bettvolumen separat gemessen und sind in den Tabellen 1 bis 4 aufgeführt.

### Vergleichsbeispiel 1

200 ml des schwach basischen Anionenaustauschers Amberlite IRA 92 adsorbieren 97 g APFOS. Das Waschwassereluat des beladenen Ionenaustauschers beträgt 5 ppm PFOS. Die Eluierung mit 1 Mol/l wäßriger Ammoniaklösung zeigt eine Peak-Konzentration von 76 000 ppm.

### Vergleichsbeispiel 2

200 ml des mittelstark basischen Anionenaustauschers Amberlite IRA 67 adsorbieren 141 g APFOS. Das Waschwassereluat des beladenen Ionenaustauschers hat eine PFOS-Konzentration von 18 ppm. Die Peak-Konzentration beträgt 16 000 ppm.

**Tabelle 1**

| Vergleichsbeispiel 1 | | Vergleichsbeispiel 2 | |
|---|---|---|---|
| Amberlite IRA 92 (schwachbasisch) | | Amberlite IRA 67 (mittelstark basisch) | |
| Bett-volumen | APFOS-Konzentration [ppm] | Bett-volumen | APFOS-Konzentration [ppm] |
| 0,44 | 22 000 | 0,43 | 260 |
| 1,23 | 60 000 | 1,36 | 1 700 |
| 2,56 | 76 000 | 2,12 | 14 000 |
| 3,58 | 24 000 | 3,07 | 16 000 |
| 4,64 | 12 000 | 4,17 | 13 000 |

### Beispiel 1

Die Eluierung von 220 ml Amberlite IRA 67 erfolgt mit 0,5 Mol/l NH₃ in Methanol, hergestellt aus Methanol und 28gew.%iger wäßriger NH₃-Lösung. Die APFOS-Konzentration im Eluat ist um ein Vielfaches größer als im Vergleichsbeispiel 2 ohne Methanol. Sie beträgt 140 000 ppm.

### Beispiel 2

220 ml Amberlite IRA 67 werden mit 1 Mol/l NH₃, hergestellt aus Methanol und 28gew.%iger wäßriger NH₃-Lösung, mit 5 Bettvolumina quantitativ eluiert. Die Peak-Konzentration beträgt 250 000 ppm.

### Beispiel 3

220 ml Amberlite IRA 67 werden mit 2 Mol/l NH₃, hergestellt aus Methanol und 28gew.%iger wäßriger NH₃-Lösung, bereits mit ungefähr 2 bis 3 Bettvolumina quantitativ eluiert. Die Peak-Konzentration beträgt 300 000 ppm.

**Tabelle 2**

| Beispiel 1 | | Beispiel 2 | | Beispiel 3 | |
|---|---|---|---|---|---|
| 0,5 Mol/l NH₃ | | 1,0 Mol/l NH₃ | | 2,0 Mol/l NH₃ | |
| Bett-volumen | APFOS-Konzentration [ppm] | Bett-volumen | APFOS-Konzentration [ppm] | Bett-volumen | APFOS-Konzentration [ppm] |
| 0,61 | 66 | 0,67 | 11 000 | 0,52 | 48 000 |
| 1,55 | 120 000 | 1,47 | 180 000 | 1,61 | 300 000 |
| 2,78 | 140 000 | 2,42 | 250 000 | 2,14 | 260 000 |
| 3,92 | 140 000 | 3,60 | 240 000 | 3,20 | 3 200 |
| 4,65 | 140 000 | 5,0 | 13 000 | 4,47 | 32 |

### Beispiel 4

240 ml Amberlite IRA 67 werden mit einer Methanollösung, enthaltend 0,9 Mol/l wäßriges NH₃ und 1,1 Mol/l NaOH, mit ungefähr 2 bis 3 Bettvolumina quantitativ eluiert und zeigten eine sehr hohe Peak-Konzentration von 340 000 ppm.

**Tabelle 3**

| Bett-volumen | APFOS-Konzentration [ppm] |
|---|---|
| 0,61 | 71 000 |
| 1,76 | 340 000 |
| 2,77 | 93 000 |
| 3,76 | 2 100 |
| 5,02 | 59 |

### Beispiel 5

220 ml Amberlite IRA 92 (schwachbasisch) werden mit 1,0 Mol/l NH₃, hergestellt aus Methanol und 28gew.%iger wäßriger NH₃-Lösung, mit deutlichem Maximum eluiert. Die APFOS-Konzentration im Eluat ist um ein Vielfaches höher als im Vergleichsbeispiel 1 ohne Methanol.

**Tabelle 4**

| Bett-volumen | APFOS-Konzentration [ppm] |
|---|---|
| 0,59 | 1 000 |
| 1,66 | 140 000 |
| 2,37 | 260 000 |
| 3,73 | 24 000 |
| 5,01 | 1 100 |

## Patentansprüche

1. Verfahren zur Rückgewinnung fluorierter Emulgatoren aus schwach bis mittelstark basischen Anionenaustauscherharzen mit mindestens einem Ammoniak enthaltenden und mit Wasser mischbaren organischen Lösungsmittel, das einen Siedepunkt unterhalb 150 °C aufweist.

2. Verfahren nach Anspruch 1, **dadurch gekennzeichnet, daß** der Emulgator ein monovalentes Salz einer Perfluoralkansäure, insbesondere der Perfluoroctansäure, ist.

3. Verfahren nach Anspruch 1 oder 2, **dadurch gekennzeichnet, daß** das Lösungsmittel oder dessen Komponenten einen Siedepunkt unterhalb 110 °C aufweisen.

4. Verfahren nach einem oder mehreren der Ansprüche 1 bis 3, in denen das Lösungsmittel Methanol ist.

5. Verfahren nach einem oder mehreren der Ansprüche 1 bis 4, **dadurch gekennzeichnet, dass** der Emulgator vom Anionenaustauscher eluiert und wiederverwendet wird.

## Claims

1. Process for the recovery of fluorinated emulsifiers from weakly to moderately basic anion exchanger resins using at least one ammonia-containing, water-miscible organic solvent which has a boiling point of below 150°C.

2. Process according to Claim 1, **characterized in that** the emulsifier is a monovalent salt of a perfluoroalkanoic acid, in particular of perfluorooctanoic acid.

3. Process according to Claim 1 or 2, **characterized in that** the solvent or its components have a boiling point of below 110°C.

4. Processes according to one or more of Claims 1 to 3, in each of which the solvent is methanol.

5. Process according to one or more of Claims 1 to 4, **characterized in that** the emulsifier is eluted off the anion exchanger and reused.

## Revendications

1. Procédé pour la récupération d'émulsifiants fluorés à partir de résines échangeuses d'anions faiblement à moyennement basiques avec au moins un solvant organique contenant de l'ammoniaque et miscible à l'eau, qui présente un point d'ébullition inférieur à 150°C.

2. Procédé selon la revendication 1, **caractérisé en ce que** l'émulsifiant est un sel monovalent d'un acide perfluoroalcanoïque, en particulier de l'acide perfluorooctanoïque.

3. Procédé selon les revendications 1 ou 2, **caractérisé en ce que** le solvant ou ses composants présentent un point d'ébullition inférieur à 110°C.

4. Procédé selon l'une ou plusieurs des revendications 1 à 3, dans lequel le solvant est le méthanol.

5. Procédé selon l'une ou plusieurs des revendications 1 à 4, **caractérisé en ce que** l'émulsifiant est élué par l'échangeur d'anions et est réutilisé.
